(19)

**Europäisches Patentamt**

**European Patent Office**

**Office européen des brevets**

(11)  **EP 3 812 753 B1**

(12)  **EUROPEAN PATENT SPECIFICATION**

(45) Date of publication and mention of the grant of the patent:
**29.11.2023  Bulletin 2023/48**

(21) Application number: **19205160.5**

(22) Date of filing: **24.10.2019**

(51) International Patent Classification (IPC):
**$G01N\ 25/18$** [(2006.01)]  **$G01N\ 33/22$** [(2006.01)]

(52) Cooperative Patent Classification (CPC):
**G01N 25/18; G01N 33/225**

(54) **DETERMINATION OF GAS-SPECIFIC PARAMETERS FROM HEAT TRANSFER IN THE TEMPERATURE JUMP REGIME**

BESTIMMUNG VON GASSPEZIFISCHEN PARAMETERN ANHAND DER WÄRMEÜBERTRAGUNG IM TEMPERATURSPRUNGBEREICH

DÉTERMINATION DE PARAMÈTRES SPÉCIFIQUES AUX GAZ À PARTIR D'UN TRANSFERT DE CHALEUR DANS LE RÉGIME DE SAUT DE TEMPÉRATURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.04.2021  Bulletin 2021/17**

(73) Proprietor: **Sensirion AG**
**8712 Stäfa (CH)**

(72) Inventors:
- **Rüegg, Andreas**
  **8712 Stäfa (CH)**
- **Hornung, Mark**
  **8712 Stäfa (CH)**

(74) Representative: **Detken, Andreas**
**Isler & Pedrazzini AG**
**Giesshübelstrasse 45**
**Postfach 1772**
**8027 Zürich (CH)**

(56) References cited:
**EP-A1- 2 629 084      EP-A1- 3 617 696**
**DE-A1-102014 008 284**

- ZHANG CHENGBIN ET AL: "Temperature jump at rough gas-solid interface in Couette flow with a rough surface described by Cantor fra", INTERNATIONAL JOURNAL OF HEAT AND MASS TRANSFER, ELSEVIER, AMSTERDAM, NL, vol. 70, 30 November 2013 (2013-11-30), pages 322-329, XP028668972, ISSN: 0017-9310, DOI: 10.1016/J.IJHEATMASSTRANSFER.2013.10.080

**Description**

TECHNICAL FIELD

The present invention relates to a method for determining a gas-specific parameter of an arbitrary gas, in particular a combustion-related parameter of a fuel gas or a heat capacity parameter. The invention further relates to a sensor device that is configured to carry out the method of the invention, and to a corresponding computer program product.

PRIOR ART

In many applications it is desired to determine combustion-related parameters of a fuel gas, like specific heat of combustion, Wobbe index or methane number, for instance, for controlling a combustion process or for determining the amount of energy delivered by a utility to a customer in order to enable billing based on the amount of delivered energy rather than on the volume of the delivered gas.

It is known to determine combustion-related parameters of fuel gases by determining their composition by gas chromatography. The required apparatuses are expensive, and such measurements are time-intensive. No real-time determination of combustion-related parameters is possible in this manner.

It is also known to determine the composition of a fuel gas using a so-called multi-gas analyzer, as available, for instance, from UNION Instruments GmbH, Karlsruhe, Germany. Also these instruments are relatively complex and expensive.

Another measurement principle is based on stoichiometric combustion of the fuel gas, as laid down in the standard ASTM D4891-13(2018). Also this method is relatively complex.

It has been suggested in the art to determine combustion-related parameters by employing correlations with physical gas parameters like density and thermal conductivity etc. In such methods, the combustion-related parameters are determined indirectly from physical parameters that can be more easily measured.

EP2015056A1 suggest determining a combustion-related parameter of a gas by employing a correlation between the combustion-related parameter and the ratio $c_p/\lambda$ of heat capacity and thermal conductivity. The ratio $c_p/\lambda$ is determined using a CMOS hot wire anemometer that is subjected to a constant mass flow. The constant mass flow is created using a sonic nozzle. This method is disadvantageous in that a relatively high pressure difference across the sonic nozzle is required.

Other methods for determining combustion-related parameters by employing correlations with physical parameters are disclosed, e.g., in WO2015075278, EP3153854A1, EP2574918A1, EP2806271A1, US5311447, DE10122039A1, DE2928739A, DE4118781A1, EP0715169A1 or EP1265068A1. All these methods also require a flow of the gas through some structure.

EP3502687 discloses a method of determining a combustion-related parameter by employing correlations with a thermal conductivity parameter, a heat capacity parameter, and a natural-convection parameter.

EP3367087A2 discloses methods and sensor devices for determining a thermal capacity of a gas. In one embodiment, the sensor device comprises a heater bridge and two sensing bridges on both sides of the heater bridge, the sensing bridges being arranged at different distances from the heater bridge. The different distances can be used to perform differential measurements in order to eliminate the effect of thermal transitions between the gas and the respective bridge.

EP2629084A1 discloses a thermal conductivity sensor comprising a wire-shaped heating element and two wire-shaped temperature sensing elements at different distances from the heating element. The distances are sufficiently small that gas pressure can be determined in addition to gas composition.

DE102014008284A1 discloses a volumetric flow sensor comprising a heater element and two temperature sensors arranged at different distances from the heater element. A medium is heated by the heater element, and the points in time are determined when a maximum in the temperature sensors occurs. The volume flow rate of the medium is determined based on the difference between these points in time. The distance between the heater and one of the temperature sensors is less than 50 $\mu$m.

EP3617696A1, which belongs to the state of the art under Art. 54(3) EPC, discloses a thermal conductivity sensor comprising a plurality of sensor wires. One of the wires serves as a heat source. At least two of the wires are spaced at different distances from the heat source and serve as temperature sensors. The distance between the heater wire and one of the temperature sensor wires is 50 $\mu$m.

SUMMARY OF THE INVENTION

It is an object of the present invention to provide a method for determining a gas-specific parameter that can be easily implemented in a highly accurate manner using a compact sensor device while not requiring that a flow of the gas is created.

This object is achieved by a method according to claim 1. Further embodiments of the invention are laid down in the dependent claims.

According to the invention, two independent heat transfer parameters of the gas are determined. In order to determine two independent heat transfer parameters, the present invention proposes to carry out two independent measurements of heat transfer through the gas across two heat transfer structures of the sensor element, the heat transfer structures being characterized by different dimensions across which the heat transfer takes place. Preferably, these measurements are carried out in a stationary state in which the temperature distribution in the sensor element is essentially time independent. The gas preferably has essentially zero flow, i.e., any gas flow, if present at all, is preferably small enough to not influence the measurements results.

The heat transfer parameters that are determined according to the present invention characterize the heat transfer through the gas while it is in contact with the sensor element. In the present invention, the heat transfer structures that define the length scale of the heat transfer are microstructures that are sufficiently small that the mean free path length of the gas particles (i.e. the gas atoms or molecules) cannot be neglected in comparison to this length scale, even at pressures in the range of 0.1 to 10 bar, e.g. at a standard pressure of 1 bar. The effective thermal conductivity of the gas in the microstructures thereby becomes dependent on the pressure of the gas and on the characteristic dimension of the heat transfer structure. This effect is called the Knudsen effect. It can be characterized by the so-called Knudsen number

$$Kn = \frac{\ell}{R_c}$$

where $\ell$ designates the mean free path length of the gas particles, and where $R_c$ designates the characteristic dimension of the heat transfer structure across which the heat transfer is effected. The most well-known application of the Knudsen effect is the Pirani pressure sensor. Rather than trying to compensate for the Knudsen effect, the present invention makes use of the Knudsen effect. In particular, at least one of the heat transfer parameters that are determined in the present invention is affected by the Knudsen effect and thus reflects the fact that the Knudsen number is not infinitely small.

The Knudsen effect is discussed in detail in the following review papers, to which reference is made:

[1] Devienne, F.M., "Low Density Heat Transfer", Advances in Heat Transfer (1965 Jan 1) 2: 271-356.
[2] Springer, George S., "Heat Transfer in Rarefied Gases", Advances in Heat Transfer (1971 Jan 1) 7: 163-218.

Based on the heat transfer parameters, the gas-specific parameter is determined. In some embodiments, the gas is a fuel gas, and the gas-specific parameter is a combustion-related parameter. The term "combustion-related parameter" is to be understood as relating to any parameter that characterizes the combustion properties of the fuel gas, in particular, its energy content. Well-known examples of combustion-related parameters are the heat of combustion per unit mass or per unit volume at some predefined standard conditions, the Wobbe index, and the methane number.

In other embodiments, the gas-specific parameter can be another parameter that is characteristic for the gas. For instance, the gas-specific parameter can be a heat capacity parameter, in particular, its volumetric heat capacity at constant pressure or at constant volume.

The heat transfer regime in which $0.001 \leq Kn \leq 0.1$ is often called the temperature jump regime. In the present invention, the characteristic dimensions of the heat transfer structures are preferably chosen such that for at least one of these structures the heat transfer is in the temperature jump regime. In other words, according to the invention, at least one of the first and second characteristic dimensions of the heat transfer structures is between 10 and 1'000 times the mean free path length.

A characteristic length scale in the temperature jump regime is the temperature jump length. In the present invention, the first heat transfer parameters is a temperature jump length parameter that is indicative of the temperature jump length.

In the temperature jump regime ($0.001 \leq Kn \leq 0.1$), the temperature distribution at the interface between the gas and a wall can be described by the following equation:

$$T_W - T_K = -g \frac{\partial T}{\partial n}$$

where $T_W$ is the temperature of the wall at the interface, $T_K$ is the temperature that the gas would have if the temperature gradient $\frac{\partial T}{\partial n}$ perpendicular to the wall that is present at some distance from the wall was continued all the way to the wall, und $g$ designates the temperature jump length. The temperature jump length is inversely proportional to the gas pressure $p$: $g \sim 1/p$. The temperature jump length at a given pressure and for a given wall structure is a characteristic parameter of the gas. It can be calculated using the following equation:

$$g = \frac{2-\alpha}{\alpha} \frac{2\gamma}{\gamma+1} \frac{\ell}{Pr}. \qquad (1)$$

Here, $\alpha$ is the thermal accommodation coefficient, $\gamma$ is the adiabatic index, and $Pr$ is the Prandtl number.

At gas pressures in the range of 0.1 to 10 bar, typically effects caused by the temperature jump regime can be observed if the heat transfer structures have a characteristic dimension in the micrometer range. Therefore, preferably at least one of the first and second characteristic dimensions is between 1 and 20 micrometers. The second characteristic dimension is preferably different from the first characteristic sensor dimension by at least a factor of 3, preferably at least a factor of 5.

It is advantageous if the second heat transfer parameter is a thermal conductivity parameter indicative of the "normal" thermal conductivity of the gas, i.e., the thermal conductivity that would be obtained in a regime where the mean free path length in the gas is negligibly small compared to both the first and second characteristic dimensions, i.e., where the Knudsen effect can be neglected and the Knudsen number is negligibly small.

The gas-specific parameter is preferably determined using a correlation function that describes a correlation between, on the one hand, the gas-specific parameter and, on the other hand, the first and second heat transfer parameters (and possibly additional parameters that have been obtained through additional measurements).

In preferred embodiments, the thermal diffusivity of the gas is additionally taken into account. The method can then comprise:

determining a thermal diffusivity parameter indicative of a thermal diffusivity of the gas,
wherein the gas-specific parameter is determined taking into account the thermal diffusivity parameter.

Methods for determining the thermal diffusivity of a gas using a microthermal sensor are well-known in the art (see, e.g., US 6,019,505). In a preferred implementation, the thermal diffusivity parameter is determined based on a phase difference of a thermal wave.

In particular, the gas-specific parameter can be determined using a correlation function that describes a correlation between, on the one hand, the gas-specific parameter and, on the other hand, the first and second heat transfer parameters and the thermal diffusivity parameter.

In addition, it can be advantageous to determine a pressure parameter that is indicative of a pressure of the gas and/or an ambient temperature parameter that is indicative of an ambient temperature of the gas, and to take at least one of these parameters into account when determining the gas-specific parameter.

In particular, the pressure parameter can be used for determining a pressure-compensated temperature jump length parameter indicative of the temperature jump length of the gas at a reference pressure and/or for determining a pressure-compensated thermal diffusivity parameter indicative of thermal diffusivity of the gas at a reference pressure. The pressure-compensated temperature jump length parameter and/or thermal diffusivity parameter can then be used for determining the gas-specific parameter.

The ambient temperature parameter can be used to compensate for the influence of changes in ambient temperature on the results of the meaurements.

In some embodiments, the method comprises the following features in combination:

- The method comprises determining a pressure parameter indicative of a pressure of the gas;
- the first heat transfer parameter is a pressure-compensated temperature jump length parameter indicative of a temperature jump length of the gas at a reference pressure, which is determined using the pressure parameter;
- the second heat transfer parameter is a thermal conductivity parameter indicative of a thermal conductivity of the gas,
- the method comprises determining a pressure-compensated thermal diffusivity parameter indicative of a thermal diffusivity of the gas at the reference pressure, using the pressure parameter; and
- the gas-specific parameter is determined using a correlation function that describes a correlation between, on the one hand, the gas-specific parameter and, on the other hand, the pressure-compensated temperature jump length parameter, the thermal conductivity parameter, and the pressure-compensated thermal diffusivity parameter.

Such embodiments of the method have proven particularly accurate if the gas-specific parameter is a combustion-related parameter, in particular, its volumetric heat of combustion or its Wobbe index, as will be demonstrated in more detail further below.

In other embodiments, the method does not require the determination of a pressure parameter. In such embodiments, preferably a quotient of a thermal diffusivity parameter and a temperature jump length parameter is formed and used for determining the gas-specific parameter, since this quotient is essentially pressure independent. In particular, the method can comprise the following features in combination:

- The method comprises determining a thermal diffusivity parameter indicative of a thermal diffusivity of the gas;
- the first heat transfer parameter is a quotient of the thermal diffusivity parameter and a temperature jump length parameter indicative of a temperature jump length of the gas;
- the second heat transfer parameter is a thermal conductivity parameter indicative of a thermal conductivity of the gas; and
- the gas-specific parameter is determined using a correlation function that describes a correlation between, on the one hand, the gas-specific parameter and, on the other hand, the quotient and the thermal conductivity parameter.

Such embodiments of the method are particularly useful if the gas-specific parameter is a heat capacity parameter, in particular, a volumetric heat capacity parameter indicative of a volumetric heat capacity of the gas.

The present invention further provides a sensor device for determining a gas-specific parameter of a gas, the sensor device comprising a sensor element as defined above and control circuitry configured to carry out the method of the present invention.

All or part of the control circuitry can be integrated with the sensor element on a common substrate. The common substrate can be a semiconductor substrate (i.e., a common semiconductor chip), and the control circuitry and the sensor element can be manufactured in/on the semiconductor substrate, e.g., using known CMOS processing techniques. In other embodiments, the common substrate can be, e.g., a common glass substrate. In some embodiments, part of the control circuitry can be implemented on a different substrate than the sensor element. In particular, the control circuitry can comprise a separate digital signal processor or microcontroller, which can be remote from the sensor element, the signal processor or microcontroller being configured to carry out at least part of the method of the present invention, in particular, the step of determining the gas-specific parameter based on the first and second heat transfer parameters. At least part of the control circuitry can be implemented as a general-purpose computer configured to execute a corresponding computer program.

The sensor device can comprise a pressure sensor and/or an ambient temperature sensor. The control circuitry can then be configured to operate the pressure sensor to determine a pressure parameter indicative of a pressure of the gas, and/or to operate the ambient temperature sensor to determine an ambient temperature parameter indicative of an ambient temperature of the gas. The control circuitry can further be configured to take the pressure parameter and/or the ambient temperature parameter into account during determination of the gas-specific parameter.

In preferred embodiments, the sensor element is configured as follows: The sensor element comprises at least one active element for heating or cooling the gas and first and second temperature sensor elements. For instance, the active element can be a resistive heater or a Peltier element. The first temperature sensor element has a first distance from the active element, and the second temperature sensor element has a second distance from the active element. In this case the first heat transfer structure comprises the active element and the first temperature sensor element, and the second heat transfer structure comprises the active element and the second temperature sensor element. The first characteristic dimension can then be given, e.g., by the first distance, and the second characteristic dimension can be given by the second distance.

In particular, the sensor element can comprise a substrate, in which a recess or through-opening is formed. At least three bridges span the recess or through-opening. A first bridge is a heater bridge. The active element is disposed on the heater bridge. The term "heater bridge" is used for simplicity even though the active element may act to cool the first bridge. A second bridge is a first sensing bridge. The first sensing bridge is arranged parallel to the heater bridge on a first side of the heater bridge. The first temperature sensor element is disposed on the first sensing bridge. A third bridge is a second sensing bridge. The second sensing bridge is arranged parallel to the heater bridge at a second side of the heater bridge. The second temperature sensor element is disposed on the second sensing bridge. The heater bridge with the active element and the first sensing bridge with the first temperature sensor element then form the first heat transfer structure. Likewise, the heater bridge and the second sensing bridge with the second temperature sensor element then form the second heat transfer structure.

The first and second temperature sensor elements can be, e.g., thermopiles or resistive temperature sensor elements.

It goes without saying that the present invention also encompasses the use of the sensor device of the present invention in the method of the present invention.

The present invention further provides a computer program product for determining a gas-specific parameter of a gas using a sensor device comprising a sensor element as defined above, the computer program product comprising program instructions executable by at least one processor of control circuitry of the sensor device to carry out the method of the present invention. The computer program product can comprise a non-volatile data carrier that stores the program instructions. As explained above, the control circuitry can comprise more than one processor, and at least one processor can be arranged remotely from the sensor structure.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1    shows a schematic functional representation of a microthermal sensor device;
Fig. 2    shows a schematic block diagram of processing circuitry that may be used in conjunction with the present invention;
Fig. 3    shows a flow diagram for a method for determining a combustion-related parameter;
Fig. 4    shows a diagram representing a correlation between values of the heat of combustion per unit volume as determined based on temperature jump length, thermal conductivity and thermal diffusivity (vertical axis) and as determined by measurements (horizontal axis), for a plurality of natural gases;
Fig. 5    shows a diagram representing a correlation between values of the Wobbe index as determined based on temperature jump length, thermal conductivity and thermal diffusivity (vertical axis) and as determined by measurements (horizontal axis), for a plurality of natural gases; and
Fig. 6    shows a flow diagram for a method for determining a heat capacity parameter.

DESCRIPTION OF PREFERRED EMBODIMENTS

Exemplary configuration of sensor device

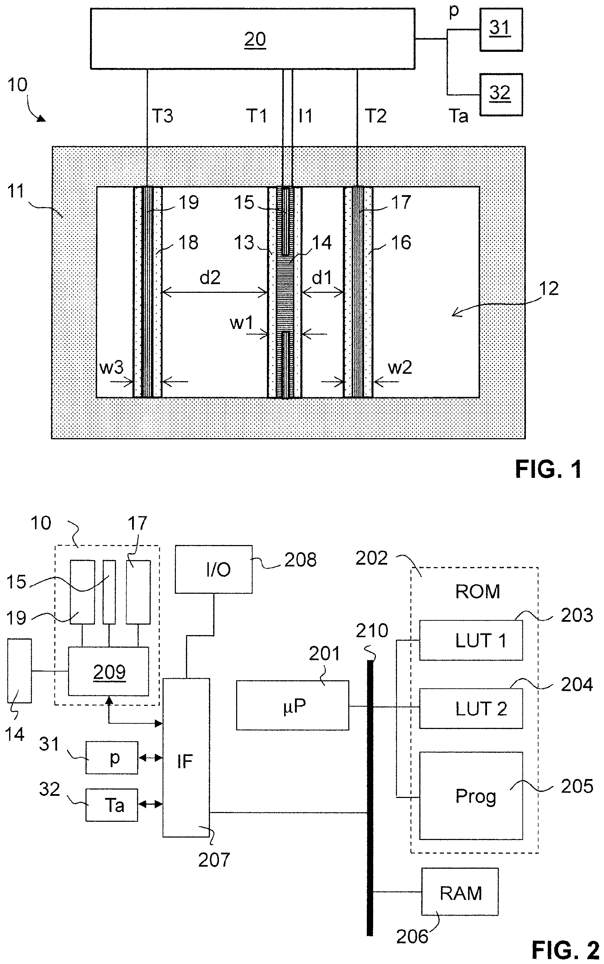

Fig. 1 shows, in a schematic manner, a microthermal sensor device according to an embodiment of the present invention. The sensor device comprises a microthermal sensor element 10 and control circuitry 20.
The sensor element 10 comprises a substrate 11, in particular a silicon substrate. The substrate 11 has an opening or recess 12 arranged therein. The sensor element 10 further comprises a plurality of separate bridges 13, 16 and 18 that span this opening or recess 32. For details, reference is made to EP 3 367 087 A2.
In the example of Fig. 1, the sensor element 10 comprises a heater bridge 13, a first sensing bridge 16 on one side of the heater bridge 13, and a second sensing bridge 18 on the other side of the heater bridge 13, each bridge spanning the recess or opening 12 and being anchored in the substrate 11. Each bridge may be formed by a plurality of dielectric layers, metal layers and/or poly-silicon layers. The metal layers or the poly-silicon layers form heater elements and temperature sensors, as will be described in more detail below. The dielectric layers may in particular comprise layers of silicon oxide and/or silicon nitride as dielectric base materials of the respective bridges. The sensing bridges 16, 18 are arranged at opposite sides of the heater bridge 13. The first sensing bridge 16 is arranged at a distance d1 to the heater bridge 13, and the second sensing bridge 18 is arranged at a different distance d2 to the heater bridge 13.
The heater bridge 13 comprises a heater element 14 and a temperature sensor 15 applied to a dielectric layer of e.g. silicon oxide. The heater element 14 and the temperature sensor 15 are electrically insulated from each other. The first sensing bridge 16 comprises a temperature sensor 17. Likewise, the second sensing bridge 18 comprises a temperature sensor 19. The temperature sensor 15 is adapted to measure the temperature of the heater bridge 13, the temperature sensor 17 is adapted to measure the temperature of the first sensing bridge 16, and the temperature sensor 19 is adapted to measure the temperature of the second sensing bridge 18. The temperature sensors can be, e.g., thermopiles or resistive elements.
The heater bridge 13 and the first sensing bridge 16 together form a first heat transfer structure. This structure has a characteristic dimension along the direction of heat transfer, the characteristic dimension being given by the distance d1 between the heater bridge 13 and the first sensing bridge 16. A second heat transfer structure is formed by the heater bridge 13 and the second sensing bridge 18, having a characteristic dimension defined by distance d2. The characteristic dimensions d1, d2 of the heat transfer structures are noticeably different. Preferably, they differ at least by a factor of 3.
At least the smaller one of the two characteristic dimensions d1, d2 is sufficiently small for the heat transfer to be governed by the temperature-jump regime, i.e., it is in the range of 10 to 1'000 times the mean free path length of the gas particles. In practice, the smaller one of the two characteristic dimensions is preferably in the range of 1 to 20 $\mu$m.

The control circuitry 20 is configured to operate the sensor element 10. The control circuitry 20 may be embodied as integrated circuitry on substrate 10, in particular, in CMOS technology. It includes circuitry for supplying a current I1 to the heater element 14 and for processing temperature signals T1, T2, T3 from the temperature sensors 15, 17 and 19, respectively. Structures of the type shown in Fig. 1 can e.g. be built using techniques such as described in EP 2 278 308 or US 2014/0208830.

Also connected to the control circuitry 20 are a pressure sensor 31 for determining a pressure parameter p, and an ambient temperature sensor 32 for determining an ambient temperature Ta of a gas in the environment of the sensor element 10.

In operation, a current I1 is provided to the heater element 14 for providing heater power, and the resulting temperatures T1, T2, and T3 are measured. At the same time, also the pressure parameter p and the ambient temperature Ta are determined. For instance, the current I1 can be controlled by the control circuitry 20 in such a manner that a predetermined temperature T1 is obtained on the heater bridge 13 once a stationary state has been established. The resulting temperatures T2 and T3 on the sensing bridges are then measured in the stationary state. A first effective thermal conductivity $\tilde{\lambda}_1$ is determined from temperature T2, and a second effective thermal conductivity $\tilde{\lambda}_2$ is determined from temperature T3, using calibration data. Calibration will be described further below. Since the temperatures T1, T2, and T3 will generally not only depend on the heater power, but also on the ambient temperature, the effective thermal conductivity values can optionally be corrected for influences of the ambient temperature Ta. Of course, more complex measurement protocols are conceivable, for instance, measurement protocols that additionally take into account the transient response of the temperature values T1, T2, and T3 to changes in heater power. Any protocol for determining two effective thermal conductivity values $\tilde{\lambda}_1$, $\tilde{\lambda}_2$ across the two different heat transfer structures of the sensor element 10 can be employed, differences between the thermal conductivity values $\tilde{\lambda}_1$, $\tilde{\lambda}_2$ reflecting the effect of the different dimensions of the heat transfer structures.

Exemplary configuration of control circuitry

Figure 2 illustrates, in a highly schematic manner, a block diagram of a possible embodiment of the processing circuitry 20. The processing circuitry comprises a processor ($\mu$P) 201, a non-volatile (e.g., Flash ROM) memory 202, and a volatile (RAM) memory 206. The processor $\mu$P communicates with the memory devices 202, 206 via a bus 210. The non-volatile memory 202 stores, inter alia, plural sets of lookup tables. In the present example, only two lookup tables LUT1 203 and LUT2 204 are illustrated. However, more lookup tables can be present. Each lookup table stores calibration data for one or more sensors. The lookup tables can correlate, for instance, the temperature values T2, T3 to the effective thermal conductivity values $\tilde{\lambda}_1$, $\tilde{\lambda}_2$, respectively. The non-volatile memory 202 further stores a machine-executable program 205 for execution in the processor $\mu$P. Via a device interface IF 207, the control device drives the heater element 14 and communicates with the various sensors 15, 17, 19, 31, and 32. An input/output interface I/O 208 enables communication to the outside world.

Determining a combustion-related parameter

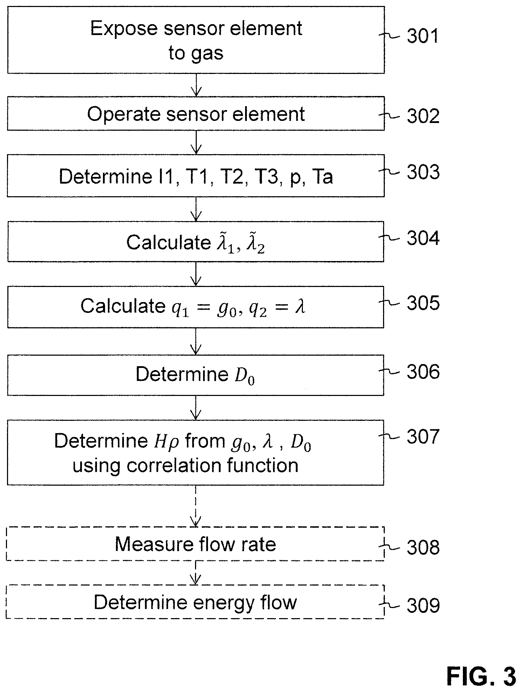

Figure 3 illustrates an exemplary flow diagram for a method of determining a combustion-related parameter using the sensor device illustrated in Figs. 1 and 2. In step 301, the sensor element 10 is exposed to the gas at essentially zero flow. In step 302, the sensor element is operated by providing a current I1 to the heater element 14, for instance providing the current in a controlled manner to cause a certain stationary-state temperature value T1. In step 303, the current I1, the temperature values T1, T2, T3 and Ta and the pressure p are determined. In step 304, the effective thermal conductivity values $\tilde{\lambda}_1$, $\tilde{\lambda}_2$ are determined from these measured values, for instance, by using look-up tables LUT1 and LUT2 (and possible further look-up tables). In step 305, two heat transfer parameters $q_1$, $q_2$ are determined from the effective thermal conductivity values $\tilde{\lambda}_1$, $\tilde{\lambda}_2$. The heat transfer parameters are the temperature jump length $g_0$ at standard pressure and the thermal conductivity $\lambda$. Their determination from the effective thermal conductivity values $\tilde{\lambda}_1$, $\tilde{\lambda}_2$ will be explained in more detail below. In step 306, thermal diffusivity $D_0$ at standard pressure is determined, as will also be discussed in more detail below. In step 307, a combustion-related parameter (e.g., the heat of combustion per unit volume, $H\rho$) is determined from $g_0$, $\lambda$, and $D_0$, which again will be described in more detail below.

Optionally, the combustion-related parameter can be used to determine an energy flow parameter for a flow of the gas through some structure. To this end, in optional step 308, the same sensor or a separate flow rate sensor is exposed to a flow of the gas, and a thermal flow measurement is carried out by methods generally known in the art. In optional step 309, an energy flow parameter is calculated from the combustion-related parameter and from the thus-determined flow rate.

Determination of temperature jump length and thermal conductivity

In the following it is assumed that the effective thermal conductivities $\tilde{\lambda}_1$, $\tilde{\lambda}_2$ that are determined in the two different heat transfer structures of the sensor element 10 can be approximatively described by the following formulae:

$$\tilde{\lambda}_1 = \frac{\lambda}{1 + \frac{g}{R_1} f_\lambda^{(1)}},$$

$$\tilde{\lambda}_2 = \frac{\lambda}{1 + \frac{g}{R_2} f_\lambda^{(2)}},$$

Here, $\lambda$ is the "ordinary" thermal conductivity of the gas, i.e., the thermal conductivity that would be measured in the normal thermal conduction regime where the mean free path of the gas particles is negligibly small as compared to the length scale across which the heat transfer takes place (i.e., $Kn \to 0$). The parameter $g$ is the temperature jump length, $R_1$ and $R_2$ are characteristic dimensions of the heat transfer process, and $f_\lambda^{(1)}$ and $f_\lambda^{(2)}$ are correction factors.

The characteristic dimensions $R_1$ and $R_2$ are not necessarily identical to the characteristic dimensions of the heat transfer structures discussed above, i.e., in the above example, $R_1$ does not necessarily correspond to d1, and $R_2$ does not necessarily correspond to d2. The reason is that the heat transfer is not only influenced by the distances d1 and d2, but also by the lengths, widths and thicknesses of the individual bridges. The parameters $R_1$, $R_2$ are typically somewhat smaller than the distances d1, d2. They can be determined experimentally by calibration measurements using a plurality of calibration gases whose temperature jump lengths and thermal conductivities are known.

The correction factors $f_\lambda^{(1)}$ and $f_\lambda^{(2)}$ generally weakly depend on the thermal conductivity $\lambda$ and can be approximated in a linear model as follows:

$$f_\lambda^{(1)} = 1 + a_1 \lambda$$

$$f_\lambda^{(2)} = 1 + a_2 \lambda$$

with slope parameters $a_1$, $a_2$. Also these slope parameters can be determined by calibration measurements using a plurality of calibration gases.

In a simple approximation, it may be possible to assume $a_1 = a_2 = 0$ and therefore $f_\lambda^{(1)} = f_\lambda^{(2)} = 1$. In this case, simple analytic expressions are obtained for $g$ and $\lambda$:

$$\lambda = \frac{\tilde{\lambda}_1 \tilde{\lambda}_2 (R_1 - R_2)}{\tilde{\lambda}_2 R_1 - \tilde{\lambda}_1 R_2},$$

$$g = \frac{R_1 R_2 (\tilde{\lambda}_1 - \tilde{\lambda}_2)}{\tilde{\lambda}_2 R_1 - \tilde{\lambda}_1 R_2}.$$

In the more general case where $a_1$ and $a_2$ are non-zero, a system of quadratic equations needs to be solved for $\lambda$ and $g$. The solution is straightforward.

The temperature jump length $g$ strongly depends on the pressure of the gas. In order to obtain a pressure-independent parameter that is characteristic of the gas, a pressure-compensated temperature jump length $g_0$ at a reference pressure $p_0$ (e.g., standard pressure of $p_0 = 1$ bar) can be determined as follows, using a measured pressure value $p$:

$$g_0 = g\frac{p}{p_0}.$$

## Determination of thermal diffusivity

The thermal diffusivity $D$, defined as $D = \lambda/(c_p\rho)$, where $c_p\rho$ is the volumetric heat capacity at constant pressure, can be determined by subjecting the heater element to an AC current and determining the phase lag of the temperatures measured by the temperature sensors 17, 19 on the sensor bridges to the phase of the temperature measured by temperature sensor 15 on the heater bridge. This procedure of determining thermal diffusivity is well known, and various modifications to this basic principle exist. Reference is made to US 6,019,505. The effect of the temperature jump regime on the measured thermal diffusivity values can be readily taken into account by calibration measurements. If the characteristic dimensions of the heat-transfer structures are not too small, the effects of the temperature jump regime on the measured thermal diffusivity values can be neglected to a good approximation.

The thermal diffusivity $D$ strongly depends on pressure, and therefore a pressure-compensated thermal diffusivity at reference pressure $p_0$ is determined as follows:

$$D_0 = D\frac{p}{p_0}.$$

## Determination of combustion-related parameters using correlation functions

Correlations between, on the one hand, combustion-related parameters and, on the other hand, the heat transfer parameters $\lambda$, $g_0$ and $D_0$ were determined as follows:
Heat transfer parameters $\lambda$, $g_0$ and $D_0$ and combustion-related parameters $H\rho$, $WI$ etc. at reference conditions $T_0 = 25$ °C and $p_0 = 1$ bar were obtained for more than 1'300 natural gases, using the database and calculation suite PPDS (Physical Property Data Services) of TUV SUD National Engineering Laboratory. The selection of gases included a wide range of gases, in particular, H gases, L gases, biogases and $H_2$-containing gases. Thermal conductivity $\lambda$ and thermal diffusivity $D_0$ of the selected gases were directly obtained from PPDS. The temperature jump length $g_0$ was determined from the mean free path length $\ell$, using the following formula:

$$g_0 = \frac{2-\alpha}{\alpha}\frac{2\gamma}{\gamma+1}\frac{\ell}{Pr}.$$

Here, $\alpha$ designates the thermal accommodation coefficient, $\gamma$ the adiabatic index, and $Pr$ the Prandtl number. Since the thermal accommodation coefficient is a quantity that depends on the material of the heat transfer structure, the assumption $\alpha = 1$ was made.

Since the mean free path length $\ell$ was not directly available from PPDS, the mean free path length $\ell$ was calculated for each gas from the viscosity $\eta$ (which was available from PPDS), using the following formula:

$$\ell = \frac{\eta}{p_0}\sqrt{\frac{\pi}{2}\frac{RT_0}{M}},$$

where $R$ is the gas constant and M is the molar mass of the gas.

The heat transfer parameters $\lambda$, $g_0$ and $D_0$ were normalized by dividing them by the corresponding values for methane ($CH_4$), obtaining three dimensionless parameters $x_1$, $x_2$ and $x_3$:

$$x_1 = \frac{\lambda}{\lambda_{CH4}},$$

$$x_2 = \frac{g_0}{g_{0,CH4}},$$

$$x_3 = \frac{D_0}{D_{0,CH4}}$$

For describing the dependence of each combustion-related parameter $y = H\rho$, $WI$ etc. on the three parameters $\tilde{\lambda}_1$, $x_2$ and $x_3$, a multilinear regression function was fitted:

$$y = \beta_0 + \beta_1 x_1 + \beta_2 x_1^2 + \beta_3 x_2 + \beta_4 x_2^2 + \beta_5 x_3 + \beta_6 x_3^2 + \beta_7 x_1 x_2 + \beta_8 x_1 x_3 + \beta_9 x_2 x_3$$

For instance, for the volumetric heat of combustion $y = H\rho$ the regression parameters in Table 1 were obtained.

Table 1: Regression parameters in MJ/m$^3$ for $y = H\rho$.

| $\beta_0$ | $\beta_1$ | $\beta_2$ | $\beta_3$ | $\beta_4$ | $\beta_5$ | $\beta_6$ | $\beta_7$ | $\beta_8$ | $\beta_9$ |
|---|---|---|---|---|---|---|---|---|---|
| 88.50 | 60.00 | 12.21 | -112.00 | -75.03 | -35.74 | -70.00 | 14.33 | -33.29 | 187.00 |

The regression function (which is an example of a correlation function) was then used for calculating the parameter $y$ from measurements of $\lambda$, $g_0$ and $D_0$, as described above, for unknown gases.

Quality of regression model

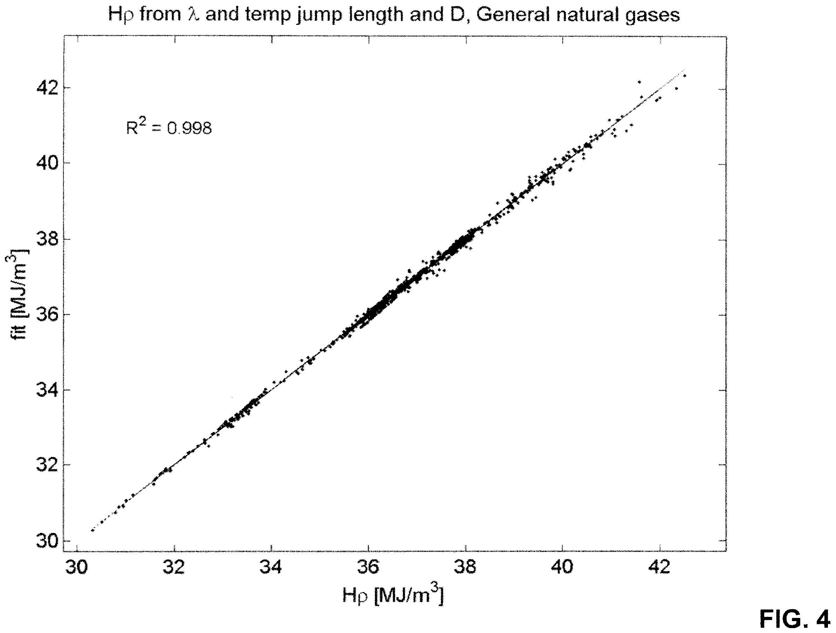

Figure 4 illustrates the quality of the correlation for the volumetric heat of combustion $H\rho$. Along the horizontal axis, the volumetric heat of combustion as obtained from PPDS is plotted. Along the vertical axis, the calculated volumetric heat of combustion, using the above regression model, is plotted. An excellent correlation is obtained, as reflected by a regression coefficient of $R^2 = 0.998$.

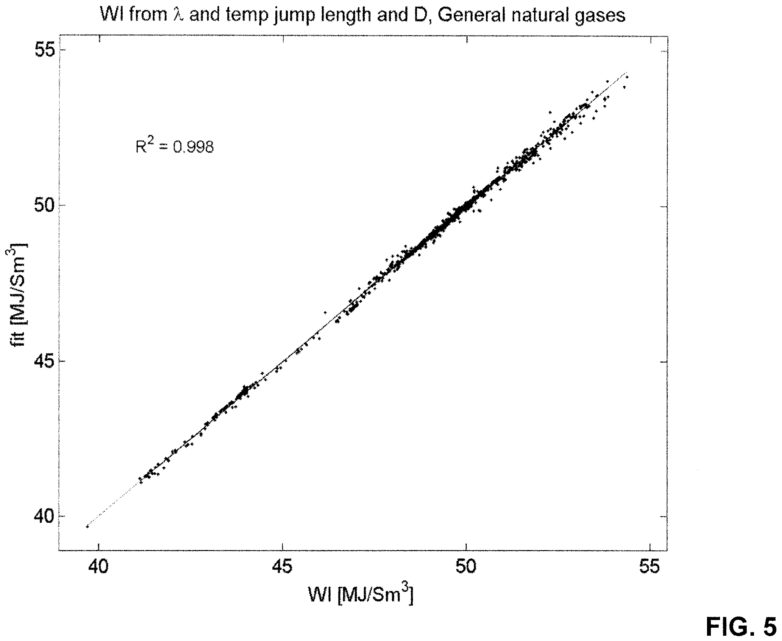

Figure 5 illustrates the correlation for the Wobbe index WI. Again, an excellent correlation is obtained.

In a similar spirit, also the correlations of other gas-specific parameters with $\lambda$, $g_0$ and $D_0$ can be used to determine such parameters, including heat capacity parameters.

In a simplified version, the thermal diffusivity is not determined, and accordingly the regression model contains less terms. Still, a satisfactory correlation can be obtained.

Absence of pressure sensor

In the above example, the temperature jump length and thermal diffusivity at standard pressure were determined, using a pressure sensor. If a pressure sensor is absent, a similar procedure can be carried out nevertheless.

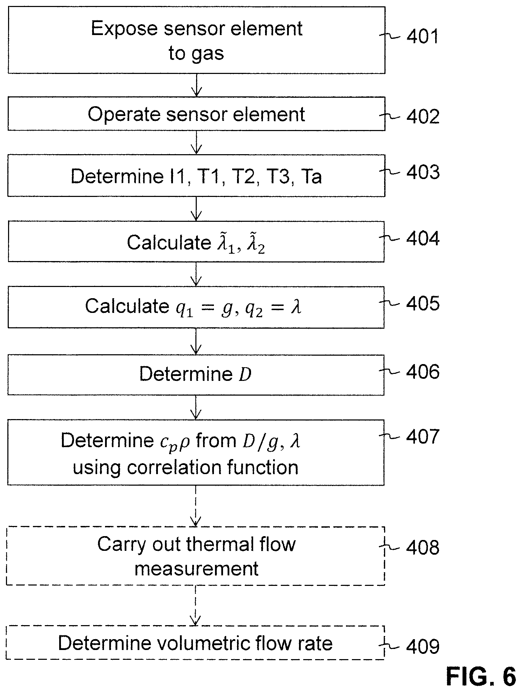

Such a method is illustrated in Fig. 6. In this example, instead of determining a combustion-related parameter, a heat capacity parameter, in particular, the volumetric heat capacity $c_p\rho$ at standard conditions, is determined. In step 401, the sensor element is exposed to an unknown gas. In step 402, the sensor element 10 is operated to determine, in step 403, the parameters I1, T1, T2, T3 and Ta. No measurement of pressure is carried out in this embodiment. In step 404, the effective thermal conductivities $\tilde{\lambda}_1$, $\tilde{\lambda}_2$ are determined, in the same manner as described above. In step 405, the parameters $g$ and $\lambda$ are calculated, again in the same manner as described above. In step 406, the thermal diffusivity $D$ is determined, again as described above.

Since both $g$ and $D$ are pressure-dependent, the pressure-independent ratio $D/g$ is formed in step 407, and the volumetric heat capacity $c_p\rho$ at standard conditions is determined, using a correlation function that describes the correlation between, on the one hand, $c_p\rho$ and, on the other hand, $D/g$ and $\lambda$.

The results of this measurement can optionally be used to determine a volumetric flow rate at standard conditions from a thermal flow measurement in an unknown gas (optional steps 308 and 309).

The correlation function can again be a multilinear regression function, similar to the one discussed above.

The same method can not only be used to determine the volumetric heat capacity $c_p\rho$ at standard conditions, but also other parameters that are not easily accessible by direct measurement, including combustion-related parameters.

Modifications

It is noted that in the above example the heater element and the temperature sensors are provided on sensing bridges that are spaced by recesses or openings, and that sensing bridges 16, 18 have equal widths w2, w3. However, the widths can also be different. The sensor element can have a different construction as long as it defines two heat transfer structures with different and sufficiently small characteristic dimensions.

LIST OF REFERENCE SIGNS

| | | | |
|---|---|---|---|
| 10 | sensor element | 32 | ambient temperature sensor |
| 11 | substrate | 201 | microprocessor |
| 12 | opening | 202 | ROM |
| 13 | heater bridge | 203 | lookup table |
| 14 | heater element | 204 | lookup table |
| 15 | temperature sensor | 205 | program data |
| 16 | sensing bridge | 206 | RAM |
| 17 | temperature sensor | 207 | device interface |
| 18 | sensing bridge | 208 | I/O interface |
| 19 | temperature sensor | 301-309 | method step |
| 20 | control circuitry | 401-409 | method step |
| 31 | pressure sensor | | |

**Claims**

1. A method for determining a gas-specific parameter ($H\rho$; $WI$; $c_p\rho$) of a gas using a sensor element (10) defining first and second heat transfer structures for causing heat transfer through the gas, the first heat transfer structure having a first characteristic dimension (d1) across which the heat transfer is effected, the second heat transfer structure having a second characteristic dimension (d2) across which the heat transfer is effected, the first and second characteristic dimensions (d1, d2) being different, at least one of the first and second characteristic dimensions (d1, d2) being between 10 and 1'000 times a mean free path length ($\ell$) in the gas, the method comprising:

   operating the sensor element (10) to cause heat transfer through the gas in contact with each of the first and second heat transfer structures;
   determining first and second heat transfer parameters ($q_1$, $q_2$) indicative of the heat transfer through the gas in contact with the first and second heat transfer structures; and
   determining the gas-specific parameter ($H\rho$; $WI$; $c_p\rho$) based on the first and second heat transfer parameters ($q_1$, $q_2$),
   **characterised in that** the first heat transfer parameter ($q_1$) is a temperature jump length parameter ($g$) that is indicative of a temperature jump length of the gas.

2. The method of claim 1, wherein the second heat transfer parameter ($q_2$) is a thermal conductivity parameter ($\lambda$) that is indicative of a thermal conductivity of the gas in a regime where the mean free path length in the gas is less than 0.001 times each of the first and second characteristic dimensions (d1, d2).

3. The method of any one of the preceding claims, wherein the gas-specific parameter ($H\rho$; $WI$; $c_p\rho$) is determined using a correlation function that describes a correlation between, on the one hand, the gas-specific parameter ($H\rho$; $WI$; $c_p\rho$) and, on the other hand, the first and second heat transfer parameters ($q_1$, $q_2$).

4. The method of any one of the preceding claims, comprising:

   determining a thermal diffusivity parameter ($D$) indicative of a thermal diffusivity of the gas,
   wherein the gas-specific parameter ($H\rho$; $WI$; $c_p\rho$) is determined taking into account the thermal diffusivity parameter ($D$),
   in particular, using a correlation function that describes a correlation between, on the one hand, the gas-specific parameter ($H\rho$; $WI$; $c_p\rho$) and, on the other hand, the first and second heat transfer parameters ($q_1$, $q_2$) and the thermal diffusivity parameter ($D$).

5. The method according to any one of the preceding claims, comprising:

   determining a pressure parameter ($p$) indicative of a pressure of the gas, and/or an ambient temperature parameter (Ta) indicative of an ambient temperature of the gas,

wherein the gas-specific parameter ($H\rho$; $WI$; $c_p\rho$) is determined taking into account the pressure parameter ($p$) and/or the ambient temperature parameter (Ta),

in particular, wherein the temperature jump length parameter ($g$) is a pressure-compensated temperature jump length parameter ($g_0$) indicative of a temperature jump length of the gas at a reference pressure ($p_0$), the pressure-compensated temperature jump length parameter ($g_0$) being calculated using the pressure parameter ($p$).

6. The method according to any one of the preceding claims,

wherein the method comprises determining a pressure parameter ($p$) indicative of a pressure of the gas,

wherein the temperature jump length parameter ($g$) is a pressure-compensated temperature jump length parameter ($g_0$) indicative of a temperature jump length of the gas at a reference pressure ($p_0$),

wherein the second heat transfer parameter ($q_2$) is a thermal conductivity parameter ($\lambda$) indicative of a thermal conductivity of the gas,

wherein the method comprises determining a pressure-compensated thermal diffusivity parameter ($D_0$) indicative of a thermal diffusivity of the gas at the reference pressure ($p_0$), and

wherein the gas-specific parameter ($H\rho$; $WI$; $c_p\rho$) is determined using a correlation function that describes a correlation between, on the one hand, the gas-specific parameter ($H\rho$; $WI$; $c_p\rho$) and, on the other hand, the pressure-compensated temperature jump length parameter ($g_0$), the thermal conductivity parameter ($\lambda$), and the pressure-compensated thermal diffusivity parameter ($D_0$),

wherein preferably the gas-specific parameter is a combustion-related parameter, in particular, a volumetric heat of combustion ($H\rho$) or a Wobbe index ($WI$).

7. The method according to any one of claims 1 to 6,

wherein the method comprises determining a thermal diffusivity parameter ($D$) indicative of a thermal diffusivity of the gas,

wherein the method comprises determining a quotient ($D/g$) of the thermal diffusivity parameter ($D$) and the temperature jump length parameter ($g$),

wherein the second heat transfer parameter ($q_2$) is a thermal conductivity parameter ($\lambda$) indicative of a thermal conductivity of the gas,.

wherein the gas-specific parameter ($H\rho$; $WI$; $c_p\rho$) is determined using a correlation function that describes a correlation between, on the one hand, the gas-specific parameter ($H\rho$; $WI$; $c_p\rho$) and, on the other hand, the quotient ($D/g$) and the thermal conductivity parameter ($\lambda$),

wherein preferably the gas-specific parameter ($H\rho$; $WI$; $c_p\rho$) is a heat capacity parameter, in particular, a volumetric heat capacity parameter ($c_p\rho$) indicative of a volumetric heat capacity of the gas.

8. A sensor device for determining a gas-specific parameter ($H\rho$; $WI$; $c_p\rho$) of a gas, the sensor device comprising:

a sensor element (10) defining first and second heat transfer structures for causing heat transfer through the gas, the first heat transfer structure having a first characteristic dimension (d1) across which the heat transfer is effected, the second heat transfer structure having a second characteristic dimension (d2) across which the heat transfer is effected, the first and second characteristic dimensions (d1, d2) being different, at least one of the first and second characteristic dimensions (d1, d2) being between 1 and 20 micrometers;

**characterised in that** the sensor device comprises control circuitry (20) configured to carry out the method of any one of the preceding claims.

9. The sensor device of claim 8, wherein the second characteristic dimension (d2) is different from the first characteristic dimension (d1) by at least a factor of 3, preferably at least a factor of 5.

10. The sensor device according to claim 8 or 9,

comprising a pressure sensor (31) and/or an ambient temperature sensor (32),

wherein the control circuitry (20) is configured to operate the pressure sensor (31) to determine a pressure parameter ($p$) indicative of a pressure of the gas, and/or to operate the ambient temperature sensor (32) to determine an ambient temperature parameter (Ta) indicative of an ambient temperature of the gas,

and wherein the control circuitry (20) is configured to take the pressure parameter (p) and/or the ambient temperature parameter (Ta) into account during determination of the gas-specific parameter ($H\rho$; $WI$; $c_p\rho$).

**11.** The sensor device of any one of claims 8 to 10,

wherein the sensor element (10) comprises at least one active element (14) for heating or cooling the gas, and wherein the sensor element (10) comprises first and second temperature sensor elements (17, 19), the first temperature sensor element (17) having a first distance from the active element (15), and the second temperature sensor element (19) having a second distance from the active element (14), the first heat transfer structure comprising the active element (14) and the first temperature sensor element (17), the second heat transfer structure comprising the active element (14) and the second temperature sensor element (19), the first characteristic dimension (d1) being given by the first distance, and the second characteristic dimension (d2) being given by the second distance.

**12.** The sensor device of claim 11, wherein the sensor element (10) comprises a substrate (11) and a recess or through-opening (12) formed in the substrate (11), at least three bridges that span the recess or through opening, a first bridge being a heater bridge (13), the active element (14) disposed on the heater bridge, a second bridge being a first sensing bridge (16), the first sensing bridge (16) being arranged parallel to the heater bridge (13) on a first side of the heater bridge (13), the first temperature sensor element (17) being disposed on the first sensing bridge (16), a third bridge being a second sensing bridge (18), the second sensing bridge (16) being arranged parallel to the heater bridge (13) at a second side of the heater bridge (13), the second temperature sensor element (19) being disposed on the second sensing bridge (18).

**13.** A computer program product comprising program instructions executable by at least one processor of the control circuitry (20) of the sensor device of any one of claims 8 to 12 to carry out the method of any one of claims 1 to 7.

**Patentansprüche**

**1.** Verfahren zur Bestimmung eines gasspezifischen Parameters ($H\rho$; $WI$; $c_p\rho$) eines Gases unter Verwendung eines Sensorelements (10), das eine erste und eine zweite Wärmeübertragungsstruktur definiert, um eine Wärmeübertragung durch das Gas zu bewirken, wobei die erste Wärmeübertragungsstruktur eine erste charakteristische Abmessung (d1) aufweist, über die die Wärmeübertragung bewirkt wird, und die zweite Wärmeübertragungsstruktur eine zweite charakteristische Abmessung (d2) aufweist, über die die Wärmeübertragung bewirkt wird, wobei die erste und die zweite charakteristische Abmessung (d1, d2) unterschiedlich sind und mindestens eine der ersten und der zweiten charakteristischen Abmessung (d1, d2) zwischen dem 10- und dem 1000-fachen einer mittleren freien Weglänge ($\ell$) im Gas beträgt, wobei das Verfahren umfasst:

Betätigung des Sensorelements (10), um eine Wärmeübertragung durch das Gas in Kontakt mit jeder der ersten und zweiten Wärmeübertragungsstrukturen zu bewirken;
Bestimmung erster und zweiter Wärmeübertragungsparameter ($q_1$, $q_2$), die die Wärmeübertragung durch das Gas in Kontakt mit den ersten und zweiten Wärmeübertragungsstrukturen anzeigen; und
Bestimmung des gasspezifischen Parameters ($H\rho$; $WI$; $c_p\rho$) auf der Grundlage der ersten und zweiten Wärmeübertragungsparameter ($q_1$, $q_2$),
**dadurch gekennzeichnet, dass** der erste Wärmeübertragungsparameter ($q_1$) ein Temperatursprunglängenparameter ($g$) ist, der für eine Temperatursprunglänge des Gases kennzeichnend ist.

**2.** Verfahren nach Anspruch 1, wobei der zweite Wärmeübertragungsparameter ($q_2$) ein Wärmeleitfähigkeitsparameter ist ($\lambda$) ist, der für eine Wärmeleitfähigkeit des Gases in einem Zustandsbereich kennzeichnend ist, in dem die mittlere freie Weglänge im Gas weniger als das 0,001-fache der jeweils ersten und zweiten charakteristischen Dimension (d1, d2) beträgt.

**3.** Verfahren nach einem der vorhergehenden Ansprüche, wobei der gasspezifische Parameter ($H\rho$; $WI$; $c_p\rho$) unter Verwendung einer Korrelationsfunktion bestimmt wird, die eine Korrelation zwischen dem gasspezifischen Parameter ($H\rho$; $WI$; $c_p\rho$) einerseits und dem ersten und zweiten Wärmeübertragungsparameter ($q_1$, $q_2$) andererseits beschreibt.

**4.** Verfahren nach einem der vorhergehenden Ansprüche, umfassend:

Bestimmung eines Temperaturleitfähigkeitsparameters ($D$), der für eine Temperaturleitfähigkeit des Gases kennzeichnend ist,

wobei der gasspezifische Parameter ($H\rho$; $WI$; $c_p\rho$) unter Berücksichtigung des Temperaturleitfähigkeitsparameters ($D$) bestimmt wird,

insbesondere unter Verwendung einer Korrelationsfunktion, die eine Korrelation zwischen einerseits dem gasspezifischen Parameter ($H\rho$; $WI$; $c_p\rho$) und andererseits dem ersten und zweiten Wärmeübertragungsparameter ($q_1$, $q_2$) und dem Parameter der Temperaturleitfähigkeit ($D$) beschreibt.

5. Verfahren nach einem der vorangehenden Ansprüche, umfassend:

Bestimmung eines Druckparameters ($p$), der für einen Druck des Gases kennzeichnend ist, und/oder eines Umgebungstemperaturparameters (Ta), der für eine Umgebungstemperatur des Gases kennzeichnend ist, wobei der gasspezifische Parameter ($H\rho$; $WI$; $c_p\rho$) unter Berücksichtigung des Druckparameters ($p$) und/oder des Umgebungstemperaturparameters (Ta) bestimmt wird,

insbesondere wobei der Temperatursprunglängenparameter ($g$) ein druckkompensierter Temperatursprunglängenparameter ($g_0$) ist, der für eine Temperatursprunglänge des Gases bei einem Referenzdruck ($g_0$) kennzeichnend ist, wobei der druckkompensierte Temperatursprunglängenparameter ($g_0$) unter Verwendung des Druckparameters ($p$) berechnet wird.

6. Verfahren nach einem der vorangehenden Ansprüche,

wobei das Verfahren die Bestimmung eines Druckparameters ($p$) umfasst, der für einen Druck des Gases kennzeichnend ist,

wobei der Temperatursprunglängenparameter ($g$) ein druckkompensierter Temperatursprunglängenparameter ($g_0$) ist, der für eine Temperatursprunglänge des Gases bei einem Referenzdruck ($g_0$) kennzeichnend ist,

wobei der zweite Wärmeübertragungsparameter ($q_2$) ein Wärmeleitfähigkeitsparameter ($\lambda$) ist, der für eine Wärmeleitfähigkeit des Gases kennzeichnend ist,

wobei das Verfahren die Bestimmung eines druckkompensierten Temperaturleitfähigkeitsparameters ($D_0$) umfasst, der für eine Temperaturleitfähigkeit des Gases bei dem Referenzdruck ($g_0$) kennzeichnend ist, und

wobei der gasspezifische Parameter ($H\rho$; $WI$; $c_p\rho$) mit Hilfe einer Korrelationsfunktion bestimmt wird, die eine Korrelation zwischen einerseits dem gasspezifischen Parameter ($H\rho$; $WI$; $c_p\rho$) und andererseits dem druckkompensierten Temperatursprunglängenparameter ($g_0$), dem Wärmeleitfähigkeitsparameter ($\lambda$), und dem druckkompensierten Temperaturleitfähigkeitsparameter ($D_0$) beschreibt,

wobei der gasspezifische Parameter vorzugsweise ein verbrennungsbezogener Parameter ist, insbesondere ein volumenbezogener Brennwert ($H\rho$) oder ein Wobbe-Index ($WI$).

7. Verfahren nach einem der Ansprüche 1 bis 6,

wobei das Verfahren die Bestimmung eines Temperaturleitfähigkeitsparameters (D) umfasst, der für eine Temperaturleitfähigkeit des Gases kennzeichnend ist,

wobei das Verfahren die Bestimmung eines Quotienten ($D/g$) aus dem Temperaturleitfähigkeitsparameter ($D$) und Temperatursprunglängenparameter ($g$) umfasst,

wobei der zweite Wärmeübertragungsparameter ($q_2$) ein Wärmeleitfähigkeitsparameter ($\lambda$) ist, der für eine Wärmeleitfähigkeit des Gases kennzeichnend ist,

wobei der gasspezifische Parameter ($H\rho$; $WI$; $c_p\rho$) unter Verwendung einer Korrelationsfunktion bestimmt wird, die eine Korrelation zwischen einerseits dem gasspezifischen Parameter ($H\rho$; $WI$; $c_p\rho$) und andererseits dem Quotienten ($D/g$) und dem Wärmeleitfähigkeitsparameter ($\lambda$) beschreibt,

wobei vorzugsweise der gasspezifische Parameter ($H\rho$; $WI$; $c_p\rho$) ein Wärmekapazitätsparameter ist, insbesondere ein volumetrischer Wärmekapazitätsparameter ($c_p\rho$), der für eine volumetrische Wärmekapazität des Gases kennzeichnend ist.

8. Sensoreinrichtung zur Bestimmung eines gasspezifischen Parameters ($H\rho$; $WI$; $c_p\rho$) eines Gases, wobei die Sensorvorrichtung umfasst:

ein Sensorelement (10), das eine erste und eine zweite Wärmeübertragungsstruktur definiert, um eine Wärmeübertragung durch das Gas zu bewirken, wobei die erste Wärmeübertragungsstruktur eine erste charakteristische Abmessung (d1) aufweist, über die die Wärmeübertragung bewirkt wird, und die zweite Wärmeübertragungsstruktur eine zweite charakteristische Abmessung (d2) aufweist, über die die Wärmeübertragung bewirkt wird, wobei die erste und die zweite charakteristische Abmessung (d1, d2) unterschiedlich sind und mindestens eine der ersten und der zweiten charakteristischen Abmessung (d1, d2) zwischen 1 und 20 Mikrometer liegt;

**dadurch gekennzeichnet, dass** die Sensorvorrichtung eine Steuerschaltung (20) umfasst, die dazu ausgebildet ist, das Verfahren nach einem der vorhergehenden Ansprüche durchzuführen.

9. Sensorvorrichtung nach Anspruch 8, wobei sich die zweite charakteristische Abmessung (d2) von der ersten charakteristischen Abmessung (d1) um mindestens einen Faktor 3, vorzugsweise um mindestens einen Faktor 5, unterscheidet.

10. Sensorvorrichtung nach Anspruch 8 oder 9,

umfassend einen Drucksensor (31) und/oder einen Umgebungstemperatursensor (32),
wobei die Steuerschaltung (20) dazu ausgebildet ist, den Drucksensor (31) zu betätigen, um einen Druckparameters (p) zu bestimmen, der für einen Druck des Gases kennzeichnend ist, und/oder den Umgebungstemperatursensor (32) zu betätigen, um einen Umgebungstemperaturparameter (Ta) zu bestimmen, der für eine Umgebungstemperatur des Gases kennzeichnend ist,
und wobei die Steuerschaltung (20) dazu ausgebildet ist, den Druckparameter (p) und/oder den Umgebungstemperaturparameter (Ta) bei der Bestimmung des gasspezifischen Parameters ($H\rho$; $WI$; $c_p\rho$) zu berücksichtigen.

11. Sensorvorrichtung nach einem der Ansprüche 8 bis 10,

wobei das Sensorelement (10) mindestens ein aktives Element (14) zum Erhitzen oder Kühlen des Gases umfasst, und
wobei das Sensorelement (10) ein erstes und ein zweites Temperatursensorelement (17, 19) umfasst, wobei das erste Temperatursensorelement (17) einen ersten Abstand von dem aktiven Element (15) aufweist und das zweite Temperatursensorelement (19) einen zweiten Abstand von dem aktiven Element (14) aufweist, wobei die erste Wärmeübertragungsstruktur das aktive Element (14) und das erste Temperatursensorelement (17) umfasst, wobei die zweite Wärmeübertragungsstruktur das aktive Element (14) und das zweite Temperatursensorelement (19) umfasst, wobei die erste charakteristische Abmessung (d1) durch den ersten Abstand gegeben ist und die zweite charakteristische Abmessung (d2) durch den zweiten Abstand gegeben ist.

12. Sensorvorrichtung nach Anspruch 11, wobei das Sensorelement (10) ein Substrat (11) und eine in dem Substrat (11) ausgebildete Ausnehmung oder Durchgangsöffnung (12), mindestens drei Brücken, die die Ausnehmung oder Durchgangsöffnung überspannen, umfasst, wobei eine erste Brücke eine Heizungsbrücke (13) ist, das aktive Element (14) auf der Heizungsbrücke angeordnet ist, eine zweite Brücke eine erste Fühlerbrücke (16) ist, wobei die erste Fühlerbrücke (16) parallel zu der Heizungsbrücke (13) an einer ersten Seite der Heizungsbrücke (13) angeordnet ist, wobei das erste Temperatursensorelement (17) auf der ersten Fühlerbrücke (16) angeordnet ist, wobei eine dritte Brücke eine zweite Fühlerbrücke (18) ist, wobei die zweite Fühlerbrücke (16) parallel zu der Heizungsbrücke (13) an einer zweiten Seite der Heizungsbrücke (13) angeordnet ist, wobei das zweite Temperatursensorelement (19) auf der zweiten Fühlerbrücke (18) angeordnet ist.

13. Computerprogrammprodukt umfassend Programmanweisungen, die von mindestens einem Prozessor der Steuerschaltung (20) der Sensorvorrichtung nach einem der Ansprüche 8 bis 12 ausgeführt werden können, um das Verfahren nach einem der Ansprüche 1 bis 7 durchzuführen.

**Revendications**

1. Méthode pour déterminer un paramètre spécifique au gaz ($H\rho$; $WI$; $c_p\rho$) d'un gaz en utilisant d'un élément capteur (10) définissant une première et une deuxième structure de transfert de chaleur pour causer un transfert de chaleur à travers le gaz, la première structure de transfert de chaleur ayant une première dimension caractéristique (d1) à travers laquelle le transfert de chaleur est effectué, la deuxième structure de transfert de chaleur ayant une deuxième dimension caractéristique (d2) à travers laquelle le transfert de chaleur est effectué, la première et la deuxième dimension caractéristique (d1, d2) étant différentes, au moins l'une de la première et de la deuxième dimension caractéristique (d1, d2) étant comprise entre 10 et 1'000 fois une longueur moyenne de libre parcours (-e) dans le gaz, la méthode comprenant:

faire fonctionner l'élément capteur (10) pour provoquer un transfert de chaleur à travers le gaz en contact avec chacune des première et deuxième structures de transfert de chaleur ;
déterminer les premier et deuxième paramètres de transfert de chaleur ($q_1$, $q_2$) reflétant le transfert de chaleur à travers le gaz en contact avec les première et deuxième structures de transfert de chaleur ; et
déterminer le paramètre spécifique au gaz ($H\rho$; $WI$; $c_p\rho$) sur la base des premier et deuxième paramètres de transfert de chaleur ($q_1$, $q_2$),
**caractérisé en ce que** le premier paramètre de transfert de chaleur ($q_1$) est un paramètre de longueur de saut de température ($g$) qui reflète une longueur de saut de température du gaz.

2. Méthode selon la revendication 1, dans laquelle le second paramètre de transfert de chaleur ($q_2$) est un paramètre de conductivité thermique ($\lambda$) reflétant une conductivité thermique du gaz dans un régime où la longueur moyenne du libre parcours dans le gaz est inférieure à 0,001 fois chacune des première et deuxième dimensions caractéristiques (d1, d2).

3. Méthode selon l'une quelconque des revendications précédentes, dans laquelle le paramètre spécifique au gaz ($H\rho$; $WI$; $c_p\rho$) est déterminé en utilisant une fonction de corrélation qui décrit une corrélation entre, d'une part, le paramètre spécifique au gaz ($H\rho$; $WI$; $c_p\rho$) et, d'autre part, les premier et deuxième paramètres de transfert de chaleur ($q_1$, $q_2$).

4. Méthode selon l'une quelconque des revendications précédentes, comprenant :

déterminer un paramètre de diffusivité thermique (D) reflétant une diffusivité thermique du gaz,
le paramètre spécifique au gaz ($H\rho$; $WI$; $c_p\rho$) étant déterminé en tenant compte du paramètre de diffusivité thermique (D),
en particulier, en utilisant une fonction de corrélation qui décrit une corrélation entre, d'une part, le paramètre spécifique au gaz ($H\rho$; $WI$; $c_p\rho$) et, d'autre part, les premier et deuxième paramètres de transfert de chaleur ($q_1$, $q_2$) et le paramètre de diffusivité thermique (D).

5. Méthode selon l'une quelconque des revendications précédentes, comprenant :

déterminer un paramètre de pression (p) reflétant une pression du gaz, et/ou un paramètre de température ambiante (Ta) reflétant une température ambiante du gaz,
le paramètre spécifique au gaz ($H\rho$; $WI$; $c_p\rho$) étant déterminé en tenant compte du paramètre de pression (p) et/ou de la température ambiante (Ta),
en particulier, le paramètre de longueur de saut de température ($g$) étant un paramètre de longueur de saut de température compensé par la pression ($g_0$) reflétant une longueur de saut de température du gaz à une pression de référence ($p_0$), le paramètre de longueur de saut de température compensé par la pression ($g_0$) étant calculé en utilisant le paramètre de pression (p).

6. Méthode selon l'une quelconque des revendications précédentes,

la méthode comprenant la détermination d'un paramètre de pression (p) reflétant la pression du gaz,
le paramètre de longueur de saut de température ($g$) étant un paramètre de longueur de saut de température compensé par la pression ($g_0$) reflétant une longueur de saut de température du gaz à une pression de référence ($p_0$),
le second paramètre de transfert de chaleur ($q_2$) étant un paramètre de conductivité thermique ($\lambda$) reflétant la conductivité thermique du gaz,
la méthode comprenant la détermination d'un paramètre de diffusivité thermique compensé par la pression ($D_0$) reflétant la diffusivité thermique du gaz à la pression de référence ($p_0$), et
le paramètre spécifique au gaz ($H\rho$; $WI$; $c_p\rho$) étant déterminé en utilisant une fonction de corrélation qui décrit une corrélation entre, d'une part, le paramètre spécifique au gaz ($H\rho$; $WI$; $c_p\rho$) et, d'autre part, le paramètre de longueur de saut de température compensé par la pression ($g_0$), le paramètre de conductivité thermique ($\lambda$) et le paramètre de diffusivité thermique compensée par la pression ($D_0$),
de préférence, le paramètre spécifique au gaz étant un paramètre lié à la combustion, en particulier une chaleur de combustion volumétrique ($H\rho$) ou un indice de Wobbe ($WI$).

7. Méthode selon l'une des revendications 1 à 6,

la méthode comprenant la détermination d'un paramètre de diffusivité thermique (D) reflétant la diffusivité thermique du gaz,

la méthode comprenant la détermination d'un quotient (D/g) du paramètre de diffusivité thermique (D) et du paramètre de longueur de saut de température (g),

le second paramètre de transfert de chaleur ($q_2$) étant un paramètre de conductivité thermique ($\lambda$) reflétant une conductivité thermique du gaz.

le paramètre spécifique au gaz ($H\rho$; $WI$; $c_p\rho$) étant déterminé à l'aide d'une fonction de corrélation qui décrit une corrélation entre, d'une part, le paramètre spécifique au gaz ($H\rho$; $WI$; $c_p\rho$) et, d'autre part, le quotient (D/g) et le paramètre de conductivité thermique ($\lambda$),

de préférence, le paramètre spécifique au gaz ($H\rho$; $WI$; $c_p\rho$) étant un paramètre de capacité thermique, en particulier un paramètre de capacité thermique volumétrique ($c_p\rho$) reflétant une capacité thermique volumétrique du gaz.

8. Dispositif de détection pour déterminer un paramètre spécifique au gaz ($H\rho$; $WI$; $c_p\rho$) d'un gaz, le dispositif de détection comprenant :

un élément capteur (10) définissant une première et une deuxième structure de transfert de chaleur pour causer un transfert de chaleur à travers le gaz, la première structure de transfert de chaleur ayant une première dimension caractéristique (d1) à travers laquelle le transfert de chaleur est effectué, la deuxième structure de transfert de chaleur ayant une deuxième dimension caractéristique (d2) à travers laquelle le transfert de chaleur est effectué, la première et la deuxième dimension caractéristique (d1, d2) étant différentes, au moins l'une de la première et de la deuxième dimension caractéristique (d1, d2) se situant entre 1 et 20 micromètres ; **caractérisé en ce que** le dispositif de détection comprend un circuit de commande (20) configuré pour effectuer la méthode de l'une quelconque des revendications précédentes.

9. Dispositif de détection selon la revendication 8, dans lequel la deuxième dimension caractéristique (d2) est différente de la première dimension caractéristique (d1) d'un facteur d'au moins 3, de préférence d'un facteur d'au moins 5.

10. Dispositif de détection selon la revendication 8 ou 9,

comprenant un capteur de pression (31) et/ou un capteur de température ambiante (32),

le circuit de commande (20) étant configuré pour faire fonctionner le capteur de pression (31) afin de déterminer un paramètre de pression (p) reflétant la pression du gaz, et/ou pour faire fonctionner le capteur de température ambiante (32) afin de déterminer un paramètre de température ambiante (Ta) reflétant la température ambiante du gaz,

le circuit de commande (20) étant configuré pour prendre en compte le paramètre de pression (p) et/ou le paramètre de température ambiante (Ta) lors de la détermination du paramètre spécifique au gaz ($H\rho$; $WI$; $c_p\rho$).

11. Dispositif de détection selon l'une des revendications 8 à 10,

dans lequel l'élément capteur (10) comprend au moins un élément actif (14) pour chauffer ou refroidir le gaz, et l'élément capteur (10) comprend un premier et un second élément capteur de température (17, 19), le premier élément capteur de température (17) ayant une première distance par rapport à l'élément actif (15), et le second élément capteur de température (19) ayant une seconde distance par rapport à l'élément actif (14), la première structure de transfert de chaleur comprenant l'élément actif (14) et le premier élément capteur de température (17), la seconde structure de transfert de chaleur comprenant l'élément actif (14) et le second élément capteur de température (19), la première dimension caractéristique (d1) étant donnée par la première distance, et la seconde dimension caractéristique (d2) étant donnée par la seconde distance.

12. Dispositif de détection de la revendication 11, dans lequel l'élément capteur (10) comprend un substrat (11) et une cavité ou une ouverture traversante (12) formée dans le substrat (11), au moins trois ponts qui enjambent la cavité ou l'ouverture traversante, un premier pont étant un pont chauffant (13), l'élément actif (14) disposé sur le pont chauffant, un deuxième pont étant un premier pont de détection (16), le premier pont de détection (16) étant disposé parallèlement au pont chauffant (13) sur un premier côté du pont chauffant (13), le premier élément capteur de température (17) étant disposé sur le premier pont de détection (16), un troisième pont étant un deuxième pont de détection (18), le deuxième pont de détection (16) étant disposé parallèlement au pont chauffant (13) sur un deuxième côté du pont chauffant (13), le deuxième élément capteur de température (19) étant disposé sur le deuxième pont de détection (18).

**13.** Produit programme d'ordinateur comprenant des instructions de programme exécutables par au moins un processeur du circuit de commande (20) du dispositif de détection de l'une des revendications 8 à 12 pour effectuer la méthode de l'une des revendications 1 à 7.

**FIG. 1**

**FIG. 2**

```
┌─────────────────────────────────┐
│     Expose sensor element       │──┐ 301
│          to gas                 │  
└─────────────────────────────────┘
              │
              ▼
┌─────────────────────────────────┐
│     Operate sensor element      │──┐ 302
└─────────────────────────────────┘
              │
              ▼
┌─────────────────────────────────┐
│   Determine I1, T1, T2, T3, p, Ta │──┐ 303
└─────────────────────────────────┘
              │
              ▼
┌─────────────────────────────────┐
│      Calculate $\tilde{\lambda}_1, \tilde{\lambda}_2$     │──┐ 304
└─────────────────────────────────┘
              │
              ▼
┌─────────────────────────────────┐
│   Calculate $q_1 = g_0, q_2 = \lambda$   │──┐ 305
└─────────────────────────────────┘
              │
              ▼
┌─────────────────────────────────┐
│        Determine $D_0$          │──┐ 306
└─────────────────────────────────┘
              │
              ▼
┌─────────────────────────────────┐
│  Determine $H\rho$ from $g_0, \lambda, D_0$  │──┐ 307
│     using correlation function  │
└─────────────────────────────────┘
              │
              ▼
┌ ─ ─ ─ ─ ─ ─ ─ ─ ─ ─ ─ ─ ─ ─ ─ ─ ┐
│        Measure flow rate        │──┐ 308
└ ─ ─ ─ ─ ─ ─ ─ ─ ─ ─ ─ ─ ─ ─ ─ ─ ┘
              │
              ▼
┌ ─ ─ ─ ─ ─ ─ ─ ─ ─ ─ ─ ─ ─ ─ ─ ─ ┐
│      Determine energy flow      │──┐ 309
└ ─ ─ ─ ─ ─ ─ ─ ─ ─ ─ ─ ─ ─ ─ ─ ─ ┘
```

**FIG. 3**

EP 3 812 753 B1

Hρ from λ and temp jump length and D, General natural gases

FIG. 4

WI from λ and temp jump length and D, General natural gases

R² = 0.998

EP 3 812 753 B1

FIG. 5

Expose sensor element
to gas — 401

Operate sensor element — 402

Determine I1, T1, T2, T3, Ta — 403

Calculate $\tilde{\lambda}_1, \tilde{\lambda}_2$ — 404

Calculate $q_1 = g$, $q_2 = \lambda$ — 405

Determine $D$ — 406

Determine $c_p\rho$ from $D/g$, $\lambda$
using correlation function — 407

Carry out thermal flow
measurement — 408

Determine volumetric flow rate — 409

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2015056 A1
- WO 2015075278 A
- EP 3153854 A1
- EP 2574918 A1
- EP 2806271 A1
- US 5311447 A
- DE 10122039 A1
- DE 2928739 A
- DE 4118781 A1
- EP 0715169 A1

- EP 1265068 A1
- EP 3502687 A
- EP 3367087 A2
- EP 2629084 A1
- DE 102014008284 A1
- EP 3617696 A1
- US 6019505 A
- EP 2278308 A
- US 20140208830 A

**Non-patent literature cited in the description**

- **DEVIENNE, F.M.** Low Density Heat Transfer. *Advances in Heat Transfer,* 01 January 1965, vol. 2, 271-356

- **SPRINGER, GEORGE S.** Heat Transfer in Rarefied Gases. *Advances in Heat Transfer,* 01 January 1971, vol. 7, 163-218